# EUROPEAN PATENT APPLICATION

(11) **EP 1 043 004 A2**
(43) Date of publication of application: **11.10.2000**
(21) Application number: 00302911.3
(22) Date of filing: 06.04.2000
(51) Int. Cl.: A61F 13/15

(54) **An absorbent article**

(30) Priority: 07.04.1999 BR 9901047
(71) Applicant: Johnson & Johnson Industria e Comercio Ltda., 05501-030 Sao Paulo, SP (BR)
(72) Inventor: Costa, Rogerio, 485 Lorena - SP (BR)
(74) Representative: Mercer, Christopher Paul

(57) **Abstract**

This invention refers to a sanitary napkin provided with a single enveloping sheet that serves to accommodating the absorbent before and after use, so as to obtain a package that prevents leakage of any fluid out of the absorbent. This objective is achieved by means of a sanitary napkin (1) comprising a liquid-permeable front layer (4), a liquid-impermeable back layer (5), an absorbent body (2) arranged between the front (4) and the back (5) layers, an enveloping sheet (16), flaps (10) extending laterally, said absorbent comprising adhesive regions (11,12) on the back layer (5), said absorbent (1) comprising an enveloping sheet (16) suitable to envelop the absorbent (1) totally, said enveloping sheet (16) comprising protection sheet (15,17) suitable to be adhered to the adhesive regions (11,12). The enveloping sheet (16) totally envelops the absorbent (1) with the flaps (10) open.

## Description

### Background of the Invention

The present invention refers to an absorbent article such as a sanitary napkin, an absorbent for urinary incontinence and the like, said absorbent being preferably disposable and provided with side tabs with adhesive regions that aid in fixing said absorbent to the wearer's panties, maintaining it in the correct position and avoiding the occurrence of leakage.

Particularly, the present invention refers to an intimate absorbent article, associated to an enveloping sheet suitable for accommodating and enveloping said absorbent before and after its use.

As known from the prior art, intimate absorbents are generally disposable and used for collecting and holding vaginal exudates, especially menstrual blood, intermenstrual secretions and also urine in the cases of incontinence.

Typically, such intimate absorbents are constituted by a body of an absorbent material, generally planar and elongate in shape, enveloped by a permeable front layer, suitable for entering in contact with the wearer's body, and an impermeable back layer in contact with her panties.

The permeable front layer is adapted for entering in contact with the wearer's pelvic region and, in general, it is made of a soft and non-irritant material. According to the present state of the art, this layer may be made of a perforated plastic film, a porous or reticulate foam, a sheet of woven or non-woven material with natural fibers (wood or cotton fibers), artificial fibers (polyester or polypropylene fibers), or else a combination of synthetic or natural fibers.

Said front layer may be of a hydrophobic material, in order to have a tendency to remain dry.

On the other hand, said impermeable pack layer has the function of preventing the absorbed and held fluid from passing to the clothes or skin of the wearer, being optionally manufactured from a sheet of polyethylene. Said back layer may be permeable to vapors and, in this case, it is either provided with small pores or made of a non-woven liquid-impermeable material.

The absorbents of the prior art usually have one or more adhesive regions on the surface of the respective impermeable back layers, especially in a central portion in the longitudinal and/or transverse direction thereof, in order to fix the absorbent in the inner part of the crotch region of the panties. Said adhesive region may be shaped as a single strip, multiple strips, in spiral, with stitches or any adequate configuration, and there is a tendency to apply the adhesive to said back layer along one or more longitudinal strips for the sake of processing ease.

Said absorbent body, in tum, may be made from any material capable of absorbing and holding bodily exudates, such as for example wood pulp, bamboo fibers, sugar-cane bagasse, corncob or corn stem, turf moss, absorbent foams or sponges, synthetic or polymeric fibers, suberabsorbent materials (which form hydrogels when they enter in contact with liquids), combinations of the above materials and others.

At present, many of the feminine intimate absorbent have flexible flaps extending to the sides, for instance, as a prolongation of one or both said front and back layers, or still separate from these layers, but associated to the absorbent. Said flaps serve to fix said absorbent to the panties, and for this purpose they bend over the edges of the crotch portion of the panties, over the outer layer thereof. Preferably, one of more regions of such flaps have stretches coated with adhesives permitting one to secure them to the panties, thus restricting the displacement of the absorbent when in use.

Said adhesive regions are preferably located on the back surface of the flap corresponding, for instance, to a prolongation of the impermeably back layer of the absorbent. As known from the prior art, some feminine absorbents, such as some designed for nocturnal use, are longer and have more than one flap on each side of the product, that is to say, each side of the absorbent has two different flaps, the absorbent having therefore four flaps. This type of product to is foreseen in the present invention.

Said adhesive-coated regions, either at the impermeable back layer of the absorbent or at the respective flaps, should be protected against any kind of contact before the absorbent is used, under pain of impairing the adhesion power of said regions or causing them to stick undesirably to inadequate points of the absorbent or clothes of the wearer, in which case the handling of the absorbent becomes difficult and eventually inadequate.

For this purpose, protection sheets are traditionally used, which are also known as "release papers", constituted by a sheet of paper covered with a layer of silicone or antiadherent resin facing the adhesive surface. Said protection sheets are only used for accommodating the absorbent prior to use.

### Description of the Prior Art

Aiming at the comfort of the wearer, intimate absorbents have been presented folded and individually packed, so that they can be carried separately and discreetly.

However, the individually packed absorbents known at present are little practical, since they require several steps of handling until it is released from its envelope and available for fixation to the wearer's panties. Usually said steps involve handling said front and back layers of the absorbent, thus impairing the hygiene as a result of contact of the wearer's hands with said front layer, which will be in close contact with the pelvic region of the wearer.

Another problem encountered in this type of absorbent is that, after it has been released from said envelope, the flaps still remain fixed to each other or to the absorbent body itself. It is then necessary to release these flaps so as to make them available for use.

### Objectives of the Invention

One of the objectives of the present invention is to provide an intimate absorbent provided with an enveloping sheet suitable for accommodating and enveloping said absorbent, the enveloping sheet being shaped and folded so as to offer also the possibility that the wearer can release the absorbent from the package in a single step and, consequently, have minimum contact with the front layer of the absorbent.

This objective is achieved by means of a feminine sanitary napkin comprising a liquid-permeable front layer, a liquid-impermeable back layer provided by adhesive regions, an absorbent body arranged between the front and back layers, flaps extending laterally, and an enveloping sheet, characterized in that said enveloping sheet totally envelopes the absorbent folded over itself, still with the flaps open.

In the prior art there are packages similar to the that of the invention. However, the absorbent body and the flaps are folded over each other, and only then they are enveloped by the enveloping sheet. These are embodiments that require great complexity in locating the protection sheets in the adhesive regions, and in folding the absorbent article. According to the present invention, the total envelopment occurs with the side flaps open (regardless of number of flaps), with less complexity in the operation of assembling the article and greater ease when opening it. The enveloping sheet of this invention tends to be larger than those of the prior art, but for this very reason it brings more safety in use for disposing used articles.

In the sense employed herein, the mention of these adhesive regions in the liquid-impermeable back layer also comprises the back of the side flaps, regardless of their being formed from the superposition of the front and back layers or not.

Preferably, even if the absorbent article of the present invention has been totally enveloped by the enveloping sheet, it may undergo later folds such as those of the flaps over the already folded absorbent, thus decreasing the total size of the article in the final configuration.

### Description of the Drawings

The present invention will now be described in greater detail with reference to the accompanying drawings, which represent a non-limitative alternative of embodiment, in which:
- figure 1 is a view of the front face of the sanitary napkin fixed to an enveloping sheet, according to a preferred embodiment of the present invention;
- figure 2 is a view of the pack face of the sanitary napkin illustrated in figure 1;
- figure 3 is a view of the front face of the sanitary napkin illustrated in figure 1, which is partly folded;
- figure 4 is a perspective view of said absorbent showing a step of folding the absorbent and of the enveloping sheet;
- figure 5 is a perspective view of the enveloping sheet containing the absorbent, illustrating another folding step;
- figure 6 is a view of the front face of the enveloping sheet partly folded;
- figure 7 is a view of the front face of the enveloping sheet completely folded, enveloping the sanitary napkin;
- figure 8 is a view of the front face of the enveloping sheet with the respective protection sheets;
- figure 9 is a perspective of the enveloping sheet illustrated ion figure 8; and
- figure 10 is a perspective view of the absorbent detached from the enveloping sheet.

### Detailed Description of the Figures

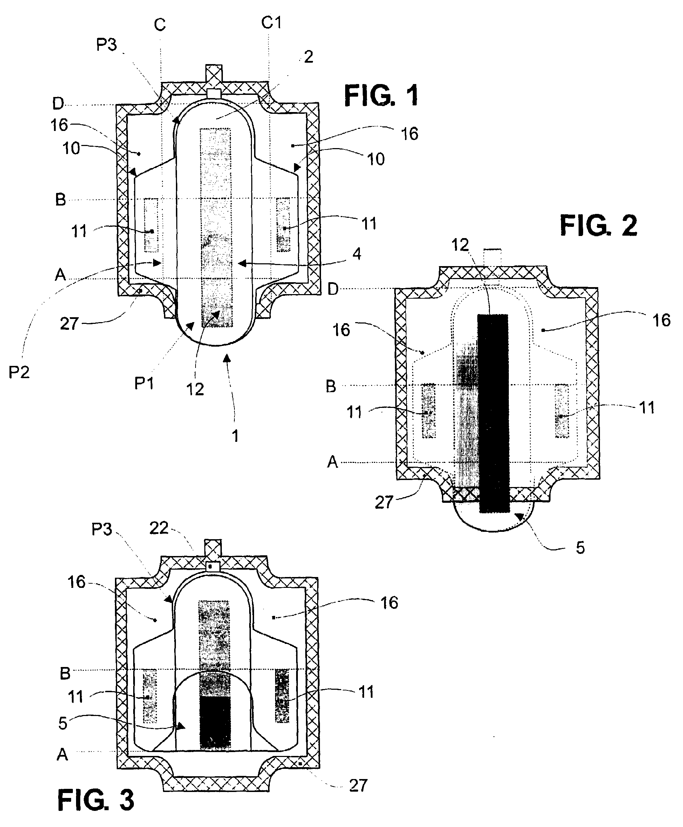

As illustrated in figures 1 - 7 and 10, the sanitary napkin 1 comprises a substantially planar body of an absorbent material 2, enveloped by a permeable front layer 4, suitable for entering in close contact with the pelvic region of the wearer and by an impermeable back layer 5 facing the respective panties (not shown).

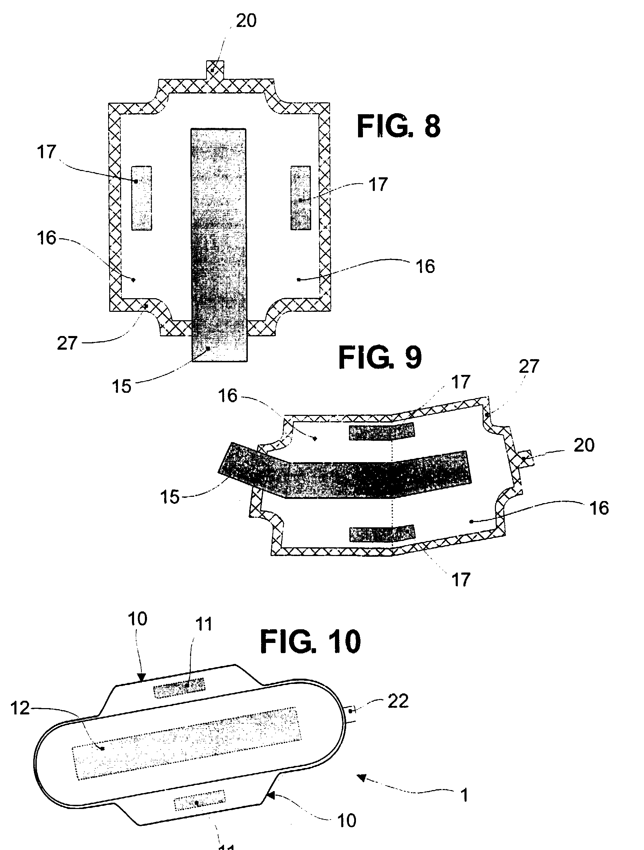

Said absorbent 1 further comprises flexible flaps 10, which extend laterally as a prolongation of the front 4 and back 5 layers, or a prolongation of only one of them, or else separated from these layers 4 and 5, but associated to the absorbent 1. In the example described, the absorbent 1 has only two flaps 10, but it could have 4 or more of them. As illustrated in figure 10, each of the pack faces of these flaps 10 has a first adhesive region 11 allowing one to fix it to the crotch portion of the panties. A second adhesive region 12 is provided in a longitudinal central portion of the back layer 5 of the absorbent 1.

The material used to achieve the adhesive regions 11 and 12 is known to those skilled in the art, being usually a permanent touch adhesive or PSA ("pressure sensitive adhesive). As already described, this material is applied in the form of a single strip, multiple strips, points or any other adequate configuration.

According to the present invention, an enveloping sheet 16 is provided, which should be conformed to the dimensions of the absorbent 1, that is to say, it should be capable of accommodating and enveloping the absorbent 1 before or after use, so as to obtain a package which prevents the leakage of any fluid retained in the absorbent 1. In other words, the enveloping sheet 16 may be as long as or longer than the absorbent 1.

As can be seen from figures 1 and 2 of the example, the enveloping sheet 16 has a shape that follows the generic shape of the contour of the absorbent 1, and end portion of the absorbent 1 defined by panel P1 projecting beyond the edge 27 of the sheet 16 when said absorbent 1 is open. The enveloping sheet 16, may be manufactured from one or more layers, being preferably manufactured from an impermeable and flexible material, so that it can be folded around the absorbent 1, and it may be a plastic film, paper, non-woven fabric, any of these materials being coated with at least some kind of resin, such as for instance antiadherent, laminate or a combination of these materials. Preferably, the sheet 16 is made from a thin impermeable plastic film such as polyethilene. As can be seen from figures 1 - 9, the edge 27 may be reinforced by a process of gripping the material, and said edge 27 may still be glued or soldered by heating when a material suitable for process is used.

As illustrated in figures 8 and 9, the envelopment sheet 16 is provided with protection sheets 15 and 17, longitudinally positioned. A pair of protection sheets 17 being suitable for temporarily protecting the adhesive regions 11, and a protection sheet 15 positioned in the central region of the sheet 16 being appropriate for temporarily protecting the second adhesive regions 12.

As known from the prior art, the protection sheets 15 and 17 should provide a temporary adhesion to the adhesive regions 11 and 12, so that the latter will not lose adhesion to the panties.

In the invention, the protection sheets 15 and 17 have two opposed faces, one that protects or adheres releasably or temporarily to the adhesive regions 12 and 11, respectively, and the other for the opposed face, which adheres definitively or non-releasably to the enveloping sheet 16.

When the absorbent 1 is fixed to the enveloping sheet 16, the back layer 5 remains in contact with said sheet 16, the front layer 4 facing the opposite side (see figure 1).

In order for the enveloping sheet 16 to envelope the absorbent 1 totally, folding lines are provided on the absorbent 1, on the flaps 10 of the absorbent 1 and simultaneously with the enveloping sheet 16, on the absorbent and simultaneously with the enveloping sheet 16, and on the enveloping sheet 16. (These folding lines may also be understood as lines along which a fold is formed, since the adjacent material is flexible and makes it possible to form a fold, without there being a physically defined line. This is also true for the other folding lines, mentioned below).

Figures 1 - 4 illustrate the folding line A, which is provided transversely in the lower portion of the absorbent 1. The folding line B is provided in the substantially middle and transverse portion of the absorbent 1 and simultaneously on the enveloping sheet 16. The folding lines C and C1 are provided longitudinally on the flaps 10 of the absorbent 1 and simultaneously on the enveloping sheet.

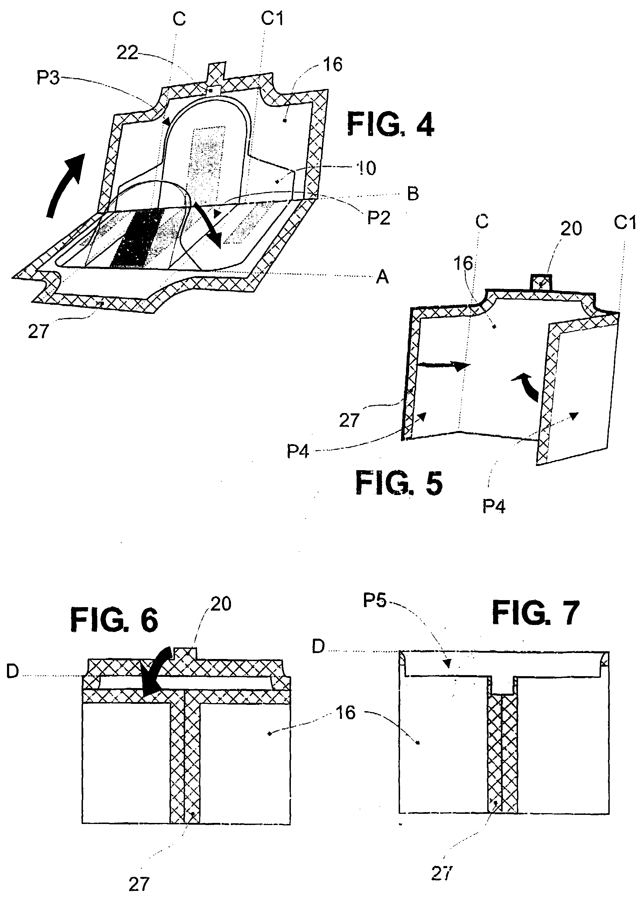

Figures 6 and 7 illustrate the folding line D, which is provided transversely in an end portion of the enveloping sheet 16.

In order to describe the steps of folding the absorbent 1 and the enveloping sheet 16, five panels P1 - P5 (see figures 1, 5, and 6) have been defined. The panels P1, P2, and P3 have approximately and preferably the same length (middle length along an imaginary longitudinal line parallel to the length of the absorbent 1).

A first panel P1 comprising only the absorbent 1 is delimited by a lower end portion of the absorbent 1 and by the folding line A.

A second panel P2 comprising the absorbent 1 and the sheet 16 is delimited by the folding lines A and B.

A third panel P3 comprising the absorbent 1 and the sheet 16 is delimited by an upper end portion of the absorbent 1 in conjunction with an upper end portion of the sheet 16 and by the folding line B.

A fourth panel P4, divided into two parts, comprises the flaps 10 of the absorbent 1 and the sheet 10, is delimited by the end side portions of the sheet 16 and by the folding lines C and C1 (see figure 5).

A fifth panel P5 comprises only the sheet 16 and is delimited by a lower end portion of the sheet 16 and by the folding line D.

The steps of folding the absorbent 1 and the enveloping sheet 16 are shown in figures 3 - 7, where one can se that at a first moment, in figures 3 and 4, the panel P1 is folded on the folding line A, in order to cover the panel P2 partly (see figure 3). In a following step, the panel P3 is folded on the folding line B, covering the panel P1 already folded in the preceding step and causing the absorbent 1 to remain totally covered by the sheet 16. It can be noted that, until this step, the flaps 10 are open.

As illustrated in figure 5, another optional step foresees the fixation of the panels P4, which are folded on the lines C and C1, causing the sheet 16 to remain with an appearance substantially like that of an open post envelop (see figure 6). A final optional step foresees the folding of the panel P5 on line D, causing the sheet 16 to envelop the absorbent 1 totally, said sheet 16 remaining with an appearance resulting from a closed post envelop (see figure 7).

Optionally, the envelopment sheet 16 may be provided with a projection 20 in a lower end portion, and this projection 20 may also comprise an adhesive surface suitable for fixing said panel P5 during the final step of folding the absorbent 1 in conjunction with the sheet 16.

It is important to mention that the invention considers other alternatives in which the absorbent is longer than the absorbent 1 illustrated in the example, in addition to the possibility of having more flaps. In this alternative equivalent embodiment, with respect to the example mentioned, the body of the absorbent may have more folds over itself, and once it is totally enveloped by the enveloping sheet, it can be folded more times, longitudinally and/or transversely, in order to acquire a smaller volume, if desired, more adequate to the use aimed at.

When there is the need for carrying the absorbent 1, the wearer can accommodate each of said absorbents 1 individually in her bag, for instance, without impairing the hygiene of said absorbent 1, since the latter is protected by the enveloping sheet 16.

When the wearer needs to use the absorbent 1, she should unfold the enveloping sheet 16, which envelopes said absorbent 1, by following these steps:

If the enveloping sheet 16 is provided with said projection 20, the wearer should first release the latter, in order to follow the steps of unfolding said sheet 16. Once said projection 20 has been released, the wearer should unfold the panels P5 and then the panels P4. By opening the panels P3 and P1 afterwards the absorbent 1 will remain available to be released from the sheet 16 (see figure 1). At this point, the absorbent 1 still remains fixed to the sheet 16 by the adhesive regions 11 and 12, which are fixed to the protection sheets 17 and 15. Optionally, in order for the absorbent 1 to be released from said sheet 16, a tab 22 is optionally provided, fixed to the impermeable back layer, or at any other point of the upper end portion of the absorbent 1. Said tab 22 is a strip of any material whatever, for example, non-woven fabric, plastics, paper, combinations thereof, etc., in the form of a strip, strap or any other that enables the wearer to pull the absorbent 1 out of the enveloping sheet 16. By the tab 22 the wearer can release the absorbent 1 from the sheet 16 with a single movement and having minimum contact with the permeable front layer 4, whereby the latter remains clean. The wearer can also release the absorbent 1 from the sheet 16 through the patent P1, which is not fixed directly to said sheet 16.

When the absorbent 1 is separated from the enveloping sheet 16, the protection sheets 15 and 17 remains fixed to the sheet 16, exposing the adhesive regions 11 and 12 to be glued to the panties. According to previous techniques, at this point the flaps 10 still remain fixed to each other or to the body of the absorbent 1 itself, which requires an additional step and causes the wearer to enter in contact with the front layer 4, thus impairing the hygiene of the absorbent 1.

After releasing the absorbent from the sheet 16, the wearer can keep said sheet 16 for enveloping the absorbent 1 later after it has been used. Since the enveloping sheet 16 is manufactured from an impermeable material, the absorbent 1 may be enveloped even after use, without the risk of leakage of fluid contained in the absorbent body 2.

Said tab 22 can be used for helping the wearer when the latter needs to release the absorbent 1 from the panties right after use of the absorbent 1, and also as an aid for handling the latter and throwing it away, or else for helping the wearer to envelope said absorbent 1 with the sheet 16.

A preferred embodiment having been described, it should be understood that the scope of the present invention embraces other possible variations, being limited only by the contents of the accompanying claims, which include the possible equivalents.

## Claims

1. An absorbent article (1) comprising:
- a liquid-permeable front layer (4);
- a liquid-impermeable back layer (5), provided with adhesive regions (11,12);
- an absorbent body (2) arranged between the front (4) and the back (5) layers;
- flaps (10) extending laterally;
- an enveloping sheet (16);
said absorbent being characterized in that said enveloping sheet (16) totally envelopes the absorbent (1) folded over itself, still with the flaps (10) open.

2. An absorbent article according to claim 1, characterized by comprising additional folds, preferably of the flaps (10), already enveloped by the enveloping sheet (16), folded over the already folded absorbent (1).

3. An absorbent article according to claim 1, characterized by comprising at least one tab (22) fixed to an end portion of said absorbent (1).

4. An absorbent article according to claim 1, characterized in that it comprises a folding line (A) provided transversely in a lower portion of said absorbent (1), said absorbent (1) and the sheet (16) comprise a folding line (B) in a substantially middle and transverse portion of the absorbent (1), the flaps (10) and the enveloping sheet (16) comprise the folding lines (C,C1) provided longitudinally with respect to said absorbent (1), the sheet (16) comprises a folding line (D) provided transversely in an end portion.

5. An absorbent article according to claim 4, characterized by comprising panels P1, P2, P3, P4, and P5, the panels P1, P2, P3, and P5 being consecutive, with the panel P2 being located between the folds A and B, preceded by P1 and followed by P3, the panel P1 being folded over P2, over which P3 is folded, P4 being divided into two opposed lateral subpanels formed by the front folds of P1, P2, and P3, the two subpanels of P4 being folded on the lines C and C1 over the already folded P1, P2, and P3. P5 being folded on line D over the two already folded subpanels P4.

6. An absorbent article according to claim 1, characterized in that the sheet (16) is impermeable.

7. An absorbent article according to claim 5, characterized in that the enveloping sheet (16) comprises a projection (20) in a lower end portion of panel P5.
